(19) **Europäisches Patentamt**
**European Patent Office**
**Office européen des brevets**

(11) **EP 4 001 432 A1**

(12) **EUROPEAN PATENT APPLICATION**

(43) Date of publication:
**25.05.2022 Bulletin 2022/21**

(21) Application number: **20207530.5**

(22) Date of filing: **13.11.2020**

(51) International Patent Classification (IPC):
**C12Q 1/6855** (2018.01)

(52) Cooperative Patent Classification (CPC):
(C-Sets available)
**C12Q 1/6855**                                        (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Miltenyi Biotec B.V. & Co. KG
51429 Bergisch Gladbach (DE)**

(72) Inventors:
• **Mendez, Adam
51429 Bergisch Gladbach (DE)**
• **Agam, Yigal
51429 Bergisch Gladbach (DE)**

(74) Representative: **Kisters, Michael Marcus
Miltenyi Biotec B.V. & Co. KG
Friedrich-Ebert Strasse 68
51429 Bergisch Gladbach (DE)**

(54) **ALGORITHMIC METHOD FOR EFFICIENT INDEXING OF GENETIC SEQUENCES USING ASSOCIATIVE ARRAYS**

(57)     The invention is directed to a Method for RNA or c-DNA sequencing of a sample comprising at least one RNA or c-DNA strand comprising the steps
a) providing a oligonucleotide having a 5' and a 3' end combined by 50 - 1000 nucleic acids that are complementary to the at least one RNA or c-DNA strand of the sample and wherein the oligonucleotide comprises at least one barcode region with 2 -20 nucleotides
b) hybridizing the oligonucleotide at the 5' and the 3' ends to complementary parts of the at least one RNA or c-DNA strand to create a padlock with a gap between the 5' and the 3' end of the padlock

c) filling the gap of the padlock with the complementary nucleotides and ligate them to generate a single strand circular template
d) multiplying the single strand circular template by a polymerase capable of rolling circle amplification into a plurality of DNA concatemers forming a rolony
e) determining the sequence of the single strand circular template into at least one read
**characterized in that** the reads are sorted by at least one hash-map key comprising a predefined sequence of 10 - 25 nucleotides.

Fig. 1

EP 4 001 432 A1

(52) Cooperative Patent Classification (CPC): (Cont.)

C-Sets
**C12Q 1/6855**

**Description**

BACKGROUND

The present invention is directed to a process for DNA/RNA sequencing aided by hash-mapping to identify target moieties or target cells from a cell sample.

"Cell sequencing" is a technology to identify genetic information obtained from a single cell by lysing the cell and conjugating the DNA of the cell with a library of polynucleotides comprising a barcode.

For this purpose, several methods of synthesising libraries of polynucleotides, isolating and lysing cells and subsequent analysing the resulting genetic information have been developed. Example for such methods are disclosed in US9388456 or US 9695468.

One major problem in all single cell sequencing methods is the amount of genetic information to be analysed since DNA or RNA may contain millions of base pairs.. Identification and indexing of genetic reads based on contiguous sequences or near-matches is therefore a common challenge within the fields of bioinformatics and next-generation sequencing (NGS). These types of operations are necessary in applications which include mapping of genomic sequences to a reference template, determining cell origins in single-cell RNA sequencing (scRNA) experiments, or classifying reads based on unique molecular identifiers (UMIs).

Ideally, the computational methods used should also scale with the size of the input data such that tasks can be performed in a time and memory efficient manner, independent of the number of elements contained within the dataset.

SUMMARY

It was therefore an object of the invention to provide an method to find and group specific genetic sequences quickly even when searching through billions of entries.

It was found that an associative array / dictionary sorting technique optionally involving a variable length segment of a UMI or barcode genetic sequence comprised of consecutive nucleotide bases A, T, G, or C and assigns a partition ID using hash-map functions to indicate a unique "key" element. Sorting of UMIs in single-cell RNA sequencing experiments is for example described in "UMI-count modeling and differential expression analysis for single-cell RNA sequencing" by Chen et al. Genome Biology (2018).

Any additional information such as entry number, genomic coordinates, or read ID which is then associated with this key element may be categorized into data buckets that can be queried using this key. While sorting subsequent reads in a given dataset, it is then no longer necessary to perform a computationally burdensome string comparison to every other sequence. Instead, we may simply check for the existence of a particular key.

If the key already exists within the dictionary, the additional information may be appended within the same bucket. If the key does not yet exist, a new partition ID may be created and the relevant information can be mapped to the new key. This technique may be further extended to allow closely resembling genetic reads, differing by only a few bases, to be grouped with the same key if similarity calculations are performed using equations such as Hamming Distance or Levenshtein Ratio.

The general use of hash-maps for sorting datasets is known, for example from US20160350394A1

The identification of barcodes for single cell genomics is described by Tambe et al. BMC Bioinformatics (2019). Further, implementation of Hamming distance to sort similar dictionary entries is disclosed in "Perfect Hamming code with a hash table for faster genome mapping" by Takenaka et al. BMC Bioinformatics (2011).

It was found that the use of hash-maps accelerates the sequencing of oligonucleotides like RNA or c-DNA samples.

Object of the invention is therefore a method for RNA or c-DNA sequencing of a sample comprising at least one RNA or c-DNA strand comprising the steps

> a) providing a oligonucleotide having a 5' and a 3' end combined by 50 - 1000 nucleic acids that are complementary to the at least one RNA or c-DNA strand of the sample and wherein the oligonucleotide comprises at least one barcode region with 2 -20 nucleotides
> b) hybridizing the oligonucleotide at the 5' and the 3' ends to complementary parts of the at least one RNA or c-DNA strand to create a padlock with a gap between the 5' and the 3' end of the padlock
> c) filling the gap of the padlock with the complementary nucleotides and ligate them to generate a single strand circular template
> d) multiplying the single strand circular template by a polymerase capable of rolling circle amplification into a plurality of DNA concatemers forming a rolony
> e) determining the sequence of the single strand circular template into at least one read

**characterized in that** the reads are sorted by at least one hash-map key comprising a predefined sequence of 10 - 25 nucleotides.

The proposed array / dictionary sorting technique can established for example in Python coding language.

BRIEF DESCRIPTION OF THE DRAWINGS

Fig. 1 shows the dictionary Sorting of Reads from Barcoded Cells. The representative diagram shows the method by which barcoded reads may be indexed and sorted using hash maps. Using the key values, entries may then be queried and returned in a time efficient manner.

Fig. 2 shows the Dictionary Sorting of Paired and Grouped Reads. Using the same hashing principles described, reads may be grouped into the same data bucket according to select substrings that contain matching sequences or complementary base pairs.

Fig. 3 shows Fig 1 Oligonucleotide padlock design. (A-B) 5'end and 3' end region that are complementary to a specific portion of a messenger RNA. (C) Priming regions used for universal amplification. (D) Barcode region used to identify the actual padlock and for selective RCA. (E) GAP of various length between the 5' and 3' end extremities created by the hybridization of the oligonucleotide creating a padlock-like structure.

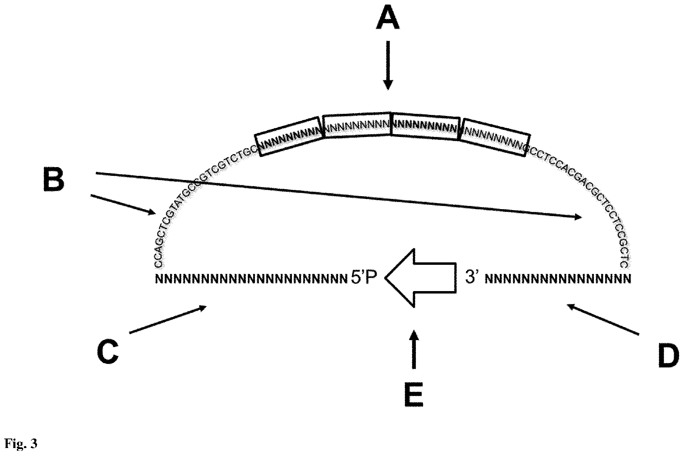

Further, Fig. 3 shows the possible priming sites (C & D) for non-selective RCA and the hybridized 5' and the 3' ends of the oligonucleotide to complementary parts of the at least one RNA or c-DNA strand. The gap (E) created between the 5' and the 3' end of the padlock may be 0 to 500, preferably less than 200.

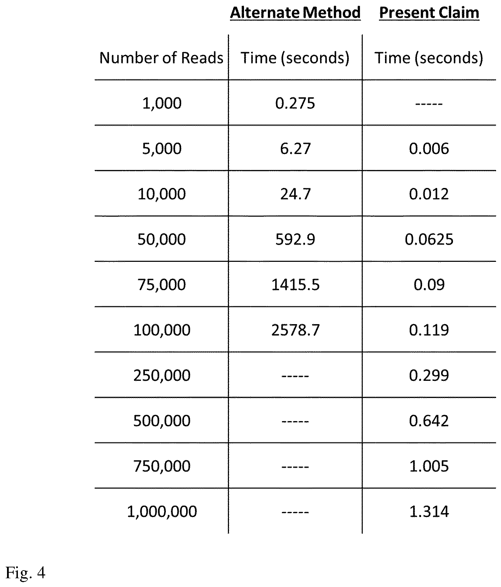

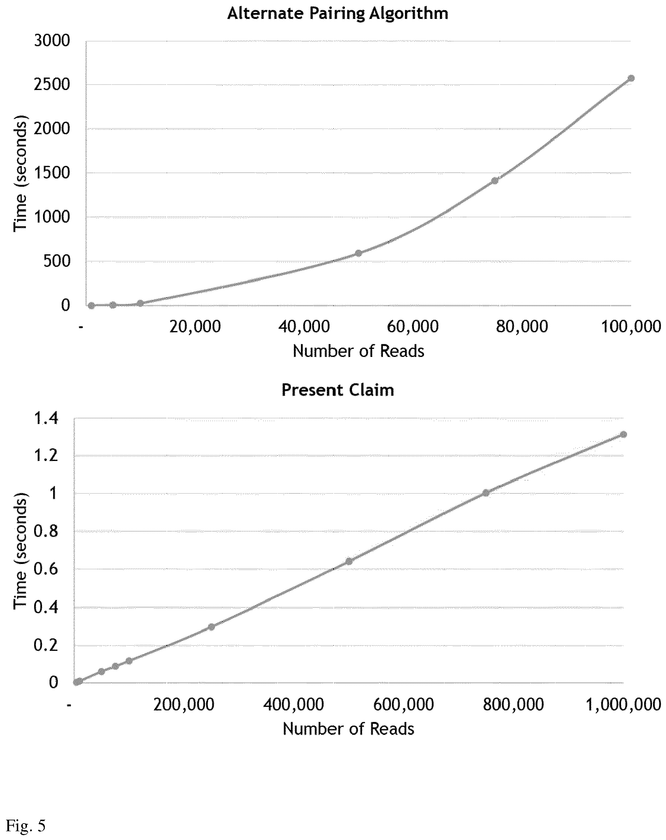

Fig. 4 and 5 demonstrate the theoretical time complexity of alternate pairing algorithms according to the number of reads which are being processed. Using a relatively slow "nested loop" approach, the computational time may take many hours when processing >250,000 reads. The hash-map algorithm method described here can be performed substantially faster, sorting millions of reads within only seconds.

DETAILED DESCRIPTION

The present invention provides an algorithmic method for efficient indexing of genomic sequences using an associative array, also known as a hash-map, symbol table, or dictionary. Using this method, genetic sequences may be sorted according to key-value pairings such that they may be used for highly time-efficient searching and grouping. This information may then be used to delineate sets of reads which originate from specific cells or that contain unique molecular identifier (UMI) and barcode sequences belonging to a particular group for next-generation sequencing (NGS) projects.

Associative arrays operate on the principle of hashing, wherein large data structures may be converted to smaller nodes, which contain linked key and value objects. Each key is assigned a unique partition identity and indexed such it may be retrieved in constant time, independent of the number of entries within the index list. By design, these types of hash-maps do not allow for duplicate keys. Therefore, any number of values associated with a particular key may be grouped into a bucket of elements having the same partition identity.

When considering the efficiency of computational algorithms, the complexity notation $O(n)$, $O(n \cdot \log n)$, $O(2^n)$, ect. is commonly used to describe how the time required to complete all necessary calculations scales with the size of the input data, being n elements. For basic string sorting algorithms, which compare a given string of m characters to all other entries within a list, a time complexity of $O(n \cdot m \cdot \log n)$ would be required, wherein $O(n \log n)$ comparisons are made in $O(m)$ time each. However, using hashing functions, the comparison time for each entry may be reduced to $O(1)$ such that an algorithm can be performed in $O(nm + n \log n)$ time. This represents a markedly faster calculation which scales more suitably with large datasets. Equation (1) represents a widely used method to define the hash of a string s comprised of n characters using a polynomial rolling hash function.

$$hash(s) = s[0] + s[1] \cdot p + s[2] \cdot p^2 + \cdots s[n-1] \cdot p^{n-1} \quad \text{modular } m$$

$$= \sum_{i=0}^{n-1} s[i] \cdot p^i \quad \text{modular } m \quad (1)$$

In this equation (1), p and m are the number of available characters and integers respectively which may be used to assign a partition ID. The hash for any substring with positional indices i through j in a string is therefore given by equation (2).

$$hash(s[i \dots j]) \cdot p^i = \sum_{k=i}^{j} s[k] \cdot p^k \quad modular\ m$$

$$= hash(s[0 \dots j]) - hash(s[0 \dots i-1]) \quad modular\ m\ (2)$$

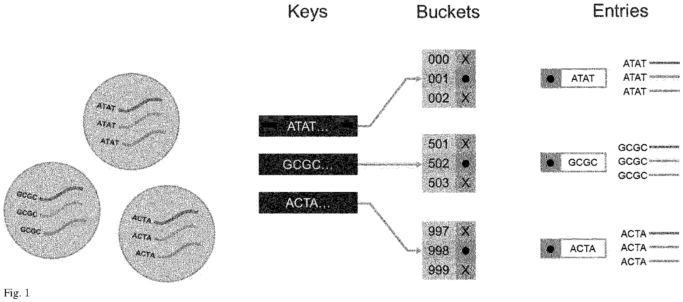

Applying this functionality within the context of NGS, sequencing reads may be converted to hash-able keys to allow for rapid sorting of genetic fragments into a dictionary data structure. The first application described here would enable efficient identification of reads originating from particular cells for reads that contain barcoded portions as in scRNA experiments (Figure 1). Here, the sequencing read may be indexed with hash functions using the full length read or the barcoded substring contained within it.

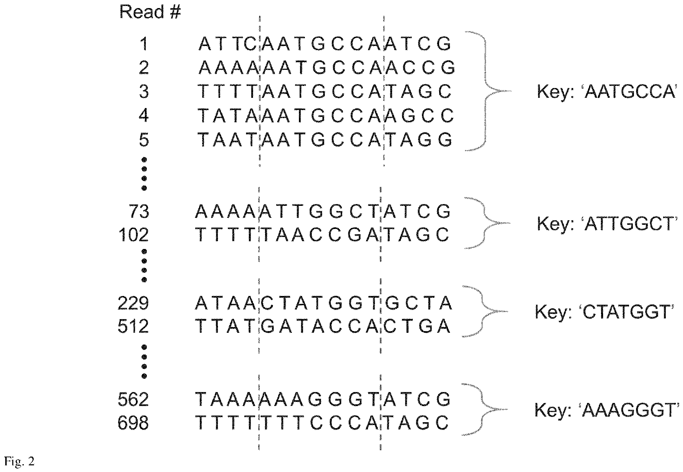

The second application of this method would enable pairing or grouping of reads according to matching substrings. For reads that contain matching sequences, a single key value may be assigned to group each read into the same data bucket. An example of this is shown in Figure 2, where a select substring from reads that contain either identical matches or complementary base pairs are used to generate dictionary keys. These keys may then be used to query any associated entry.

Additionally, these applications may be further expanded to allow grouping of reads that contain a limited number of mismatches. By including calculations which compare the similarity between strings, such as Hamming Distance, we may group reads which have a high resemblance to each other using a single key. This allowed tolerance for mismatches would help to correctly associate false-negative matches resulting from errors within a sequencing experiment.

Equation (3) describes the calculation of Hamming Distance between strings of equal length x and y. Here, dH is the number of positions where symbols differ.

$$d_H(x, y) = \sum_{i=1}^{n} \delta(x_i, y_i)\ (3)$$

Process steps

In theory step e) could be performed at the padlock instead of the single strand circular template.

In the present invention, the known gap-fill padlock technology can be modified by using padlocks that are hybridized to a specific portion of a messenger RNA directly on a section of tissue that has been fixed and permeabilized.

Padlock probes have proven very successful in polymerizing short portion of nucleic acids to which it has been hybridized to. Most padlock approaches begin by reverse transcribing the target into cDNA.

The hybridization of the padlock probe to the DNA or RNA strand is followed by a gap-fill step where a reverse polymerase fills the open section between the anchor and the extension side of the padlock from the hybridized 5' portion of the probe using the target mRNA as a guide which is then ligated to from a circular DNA molecule. Alternatively, the padlock can also be hybridized to cDNA which would require additional steps that could be bypassed by targeting the mRNA directly.

The padlock probes for which the gap has been filled and ligated to form a circular template (the probe can also be filled but ligated only further in the process). Finally, the circularized padlock probes are used as a template for rolling circle amplification (RCA) to generate a DNA strand used for sequencing. The thus obtained DNA strands are hereinafter referred to as "rolonies" or "DNA nanoballs".

The current invention is directed to a method using the gap-fill padlock where the barcode regions of the padlock oligomers may optionally be are used to obtain special information or enable further downstream processing of the obtained rolonies and/or a combination thereof.

Padlock oligonucleotides

As shown in Fig. 3, the oligonucleotide has a 5' and a 3' end recognizing a region of interest comprising around 50 - 1000 nucleic acids, preferable 50 to 200 nucleic acids and further at least one, preferably 1 to 4 barcode regions, each comprising 2 -20 nucleotides. Of course, the barcode regions have different sequences.

In the method of the invention, the single strand circular template is replicated by a polymerase capable of rolling circle amplification into a plurality of DNA concatemers forming a DNA nanoball or rolony. For this purpose, the oligonucleotide used in the present invention may comprise at least one primer region with 5 to 50 nucleotides for the rolling circle amplification.

In one embodiment of the invention, the least one primer region is located between the barcode region and the 5' and/or the 3' ends of the oligonucleotide. This embodiment is utilized if the single strand circular template shall be replicated non-selectively using oligonucleotides complementary to the padlock primer region as priming site for the rolling circle amplification polymerase.

In another embodiment of the invention, the least one barcode region is used as primer region. This embodiment is utilized if the single strand circular template is replicated selectively by using oligonucleotides complementary to the barcode region as priming site for the rolling circle amplification polymerase.

The ligation of each extremity of the padlock creating a circular molecule can occur prior to the probing or after the probing.

The padlock probes are either extracted and amplified in tube or directly amplified on fixed and permeabilized tissue using specific primers to selectively generate rolonies that will be sequenced based on the detection of the probes that actually bound to their target of interest.

The general steps of the invention are shown in Fig. 1. Here, the gap-fill padlock probe technology was used on probes that is hybridized to a specific portion of a messenger RNA directly on a section of tissue that has been fixed and permeabilized. The gap-fill region is the sequence of interest on the mRNA, and the gap between the two ends of the probe is filled by the reverse polymerase (POL) from the 5'end using the target mRNA as the guide (section A).

Following the filling of the gap, the extended hybridized padlock is probed by sequential hybridization of fluorescently labeled detector probes binding to specific internal barcodes (1-4). (section B). The visualization of the detector probes by fluorescent microscopy imaging allows for physical localization of the padlock oligonucleotide on the tissue and to determine the actual identity of the actual padlock bound to the mRNA or cDNA transcript (using padlock as fluorescence in situ hydridization probes (section C).

Finally, each extremity of the padlock are ligated creating a circular molecule. The circularized DNA is either extracted and replicated by rolling circle amplification in a tube or directly on fixed tissue using specific oligonucleotide primers binding to the barcode region to selectively generate solely rolonies that would be of interest to be sequenced (section D).

Antigen recognizing moiety

The term "antigen recognizing moiety" refers to any kind of antibody or fragmented antibody or fragmented antibody derivatives, directed against markers expressed on the cells of the cell sample. The term relates to fully intact antibodies, fragmented antibody or fragmented antibody derivatives, e.g., Fab, Fab¢, F(ab¢)2, sdAb, scFv, di-scFv, nanobodies. Such fragmented antibody derivatives may be synthesized by recombinant procedures including covalent and non-covalent conjugates containing these kind of molecules. Further examples of antigen recognizing moieties are peptide/MHC-complexes targeting TCR molecules, cell adhesion receptor molecules, receptors for costimulatory molecules, artificial engineered binding molecules, e.g., peptides or aptamers which target, e.g., cell surface molecules. Such antigen recognizing moieties antibody directed may be against antigen expressed by the biological specimens (target cells) intracellular, like IL2, FoxP3, CD154, or extracellular, like CD3, CD14, CD4, CD8, CD25, CD34, CD56, and CD133.

In a another embodiment, the probe tethered to beads or an antibody can be used to generate an array on a solid surface.

In a variant of this embodiment, the sample which is preferable a solid tissue like a fresh-frozen tissue section is placed on a covered glass slide containing the padlock forming oligonucleotide that is tethered to a bead or to an antibody. The tissue is fixed and imaged for histological purposes and permeabilized to release mRNA that can bind to adjacent padlock probes, allowing for the capture of gene expression information and later on the sequence information.

In another instance, the oligonucleotide with an antigen binding moiety binds to the regions of the sample presenting the appropriate antigen and wherein only the mRNA from the sample that are located in the vicinity of the region of interest will bind to the padlock oligonucleotide and subjected to the steps a) to e).

In one instance, the padlock are used to generate rolonies (DNA nanoballs) used in sequencing (in situ) as indicated above or can also be sequenced (ex situ). In that case, the padlocks are circularized and removed from the tissue-containing flowcell and the rolonies generated from the padlocks that have captured RNA are sequenced. When the sequenced is performed ex situ, there is less limitation in terms of read length.

In a variant of this embodiment, specific circularized padlocks can be isolated and sorted specifically when tethered to beads or released when tethered to an antibody.

In this variant, specific rolonies can be generated from the bulk released padlocks ex-vivo by using specific primers corresponding to specific barcodes to be recognized for example by the Phi29 enzyme used for RCA allowing for the selective amplification of a subset of transcripts coming from a specific region. Finally, the sequenced data are linked back to the area on the tissue where the mRNA or cDNA transcripts interacted with the padlock originally.

The padlock portion that recognizes a region of interest can also be designed to be more universal for recognizing the 3' portion of mRNA where on of the padlock binding site is composed of poly T, and a 5'side that if composed of random n-mer (random hexamer for instance). The padlock portion that recognizes a region of interest can also be designed to have modified nucleotides such as LNA to help bind the target with higher specificity.

Samples to be analysed with the disclosed method may originate from any specimen, like whole animals, organs, tissues slices, cell aggregates, or single cells of invertebrates, (e.g., Caenorhabditis elegans, Drosophila melanogaster), vertebrates (e.g., Danio rerio, Xenopus laevis) and mammalians (e.g., Mus musculus, Homo sapiens). A biological sample may have the form of a tissues slice, cell aggregate, suspension cells, or adherent cells. The cells may be living or dead.

**EXAMPLES**

A sample of human DNA was provided and subjected to the steps a to d as claimed. In step e, a varying number of reads in the range 1,000-1,000,000 were obtained and sorted either by an alternate "brute force" method as a comparison example or by the method of the present invention using hash-map keys. The brute force method utilizes nested loops within programming, wherein each entry of the barcode/UMI list is directly compared to all other entries. This results in a time complexity that is dependent on the number of times the innermost loop must be executed, which in worst cases, can be equal to $O(n^2)$. As can be seen from figure 4, the method of the present invention is substantially faster, especially while testing a greater number of reads. A time-point comparison of the pairing time according to the number of reads present also validates the theoretical time complexity of each test case, where the "brute force" nested loop approach scales exponentially with the number of reads, while the hash-map approach scales linearly. Datapoints with >250,000 reads were not obtained using the nested loop approach as the time to calculate each verges on days.

**Claims**

1. Method for RNA or c-DNA sequencing of a sample comprising at least one RNA or c-DNA strand comprising the steps

   a) providing a oligonucleotide having a 5' and a 3' end combined by 50 - 1000 nucleic acids that are complementary to the at least one RNA or c-DNA strand of the sample and wherein the oligonucleotide comprises at least one barcode region with 2 -20 nucleotides
   b) hybridizing the oligonucleotide at the 5' and the 3' ends to complementary parts of the at least one RNA or c-DNA strand to create a padlock with a gap between the 5' and the 3' end of the padlock
   c) filling the gap of the padlock with the complementary nucleotides and ligate them to generate a single strand circular template
   d) multiplying the single strand circular template by a polymerase capable of rolling circle amplification into a plurality of DNA concatemers forming a rolony
   e) determining the sequence of the single strand circular template into at least one read

   **characterized in that** the reads are sorted by at least one hash-map key comprising a predefined sequence of 10 - 25 nucleotides.

2. Method according to claim 1 **characterized in that** the oligonucleotide comprises at least one primer region with 5 to 50 nucleotides for the rolling circle amplification.

3. Method according to claim 2 **characterized in that** the least one primer region is located between the barcode region and the 5' and/or the 3' ends of the oligonucleotide.

4. Method according to any of the claims 1 to 3 **characterized in that** the single strand circular template is replicated non-selectively using oligonucleotides complementary to the padlock primer region as priming site for the rolling circle amplification polymerase.

5. Method according to any of the claims 1 to 3 **characterized in that** the least one barcode region is used as primer region.

6. Method according to claim 5 **characterized in that** the single strand circular template is replicated selectively by using oligonucleotides complementary to the barcode region as priming site for the rolling circle amplification polymerase.

7. Method according to any of the claims 1 to 6 **characterized in that** the sample comprising at least one RNA or c-DNA strand is provided from a plurality of cells or from a tissue section and wherein before or after step a), the sample is lysed or permeabilized.

8. Method according to any of the claims 1 to 7 **characterized in that** the oligonucleotide is provided with an antigen binding moiety which binds to the regions of the sample presenting the appropriate antigen and wherein only the mRNA located in the vicinity of that region is bound the padlock oligonucleotide and subjected to the steps a) to e).

9. Method according to any of the claims 1 to 8 **characterized in that** the sample is provided as tissue and the single strand circular template is replicated several time on the tissue by rolling circle amplification.

Fig. 1

Read #

| | | |
|---|---|---|
| 1 | A T T C A A T G C C A A T C G | |
| 2 | A A A A A A T G C C A A C C G | |
| 3 | T T T T A A T G C C A T A G C | Key: 'AATGCCA' |
| 4 | T A T A A A T G C C A A G C C | |
| 5 | T A A T A A T G C C A T A G G | |

| | | |
|---|---|---|
| 73 | A A A A A T T G G C T A T C G | |
| 102 | T T T T T A A C C G A T A G C | Key: 'ATTGGCT' |

| | | |
|---|---|---|
| 229 | A T A A C T A T G G T G C T A | |
| 512 | T T A T G A T A C C A C T G A | Key: 'CTATGGT' |

| | | |
|---|---|---|
| 562 | T A A A A A G G G T A T C G | |
| 698 | T T T T T T T C C C A T A G C | Key: 'AAAGGGT' |

Fig. 2

Fig. 3

|  | **Alternate Method** | **Present Claim** |
|---|---|---|
| Number of Reads | Time (seconds) | Time (seconds) |
| 1,000 | 0.275 | ----- |
| 5,000 | 6.27 | 0.006 |
| 10,000 | 24.7 | 0.012 |
| 50,000 | 592.9 | 0.0625 |
| 75,000 | 1415.5 | 0.09 |
| 100,000 | 2578.7 | 0.119 |
| 250,000 | ----- | 0.299 |
| 500,000 | ----- | 0.642 |
| 750,000 | ----- | 1.005 |
| 1,000,000 | ----- | 1.314 |

Fig. 4

Fig. 5

Europäisches
Patentamt

European
Patent Office

Office européen
des brevets

# EUROPEAN SEARCH REPORT

**Application Number**

EP 20 20 7530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2020/180813 A1 (QIAGEN SCIENCES LLC [US]; QIAGEN MANCHESTER LTD [GB]) 10 September 2020 (2020-09-10) * the whole document * | 1-9 | INV. C12Q1/6855 |
| Y | WO 2015/188192 A2 (UNIV CORNELL [US]) 10 December 2015 (2015-12-10) * the whole document * | 1-9 | |
| Y | WO 2018/114706 A1 (HOFFMANN LA ROCHE [CH]; ROCHE DIAGNOSTICS GMBH [DE] ET AL.) 28 June 2018 (2018-06-28) * the whole document * | 1-9 | |
| Y | WO 2019/117714 A1 (UMC UTRECHT HOLDING BV [NL]) 20 June 2019 (2019-06-20) * the whole document * | 1-9 | |
| Y | WO 2015/179493 A1 (CENTRILLION TECHNOLOGY HOLDING CORP; ZHOU WEI [US]) 26 November 2015 (2015-11-26) * the whole document * | 1-9 | |
| Y | WO 2015/184016 A2 (BROAD INST INC [US]; MASSACHUSETTS INST TECHNOLOGY [US]) 3 December 2015 (2015-12-03) * the whole document * | 1-9 | |
| Y | WO 2020/180659 A1 (1CELLBIO INC [US]; BRENAN COLIN J H [US] ET AL.) 10 September 2020 (2020-09-10) * the whole document * | 1-9 | |

**TECHNICAL FIELDS SEARCHED (IPC)**

C12Q

-/--

The present search report has been drawn up for all claims

| Place of search | Date of completion of the search | Examiner |
|---|---|---|
| The Hague | 19 April 2021 | Botz, Jürgen |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

**Application Number**

EP 20 20 7530

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | YU XUETING ET AL: "Mapping RNA-seq reads to transcriptomes efficiently based on learning to hash method", COMPUTERS IN BIOLOGY AND MEDICINE., vol. 116, 1 January 2020 (2020-01-01), page 103539, XP055794272, US ISSN: 0010-4825, DOI: 10.1016/j.compbiomed.2019.103539 * the whole document * | 1-9 | |
| T | WENAN CHEN ET AL: "UMI-count modeling and differential expression analysis for single-cell RNA sequencing", GENOME BIOLOGY, vol. 19, no. 1, 31 May 2018 (2018-05-31), XP055769913, DOI: 10.1186/s13059-018-1438-9 * the whole document * | | |

TECHNICAL FIELDS
SEARCHED (IPC)

The present search report has been drawn up for all claims

| Place of search | Date of completion of the search | Examiner |
|---|---|---|
| The Hague | 19 April 2021 | Botz, Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 20 20 7530

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2021

| Patent document cited in search report | | Publication date | Patent family member(s) | | Publication date |
|---|---|---|---|---|---|
| WO 2020180813 | A1 | 10-09-2020 | NONE | | |
| WO 2015188192 | A2 | 10-12-2015 | AU | 2015269103 A1 | 15-12-2016 |
| | | | CA | 2950295 A1 | 10-12-2015 |
| | | | CN | 106661631 A | 10-05-2017 |
| | | | DK | 3152331 T3 | 04-11-2019 |
| | | | EP | 3152331 A2 | 12-04-2017 |
| | | | EP | 3567122 A1 | 13-11-2019 |
| | | | ES | 2748457 T3 | 16-03-2020 |
| | | | IL | 249126 A | 30-07-2020 |
| | | | JP | 2017516487 A | 22-06-2017 |
| | | | PT | 3152331 T | 05-11-2019 |
| | | | SG | 10201810862Y A | 30-01-2019 |
| | | | SG | 11201610134P A | 27-01-2017 |
| | | | US | 2017204459 A1 | 20-07-2017 |
| | | | US | 2019345552 A1 | 14-11-2019 |
| | | | WO | 2015188192 A2 | 10-12-2015 |
| WO 2018114706 | A1 | 28-06-2018 | CN | 110062809 A | 26-07-2019 |
| | | | EP | 3559269 A1 | 30-10-2019 |
| | | | JP | 6847499 B2 | 24-03-2021 |
| | | | JP | 2020501571 A | 23-01-2020 |
| | | | US | 2019300939 A1 | 03-10-2019 |
| | | | WO | 2018114706 A1 | 28-06-2018 |
| WO 2019117714 | A1 | 20-06-2019 | CA | 3085420 A1 | 20-06-2019 |
| | | | CN | 111936634 A | 13-11-2020 |
| | | | EP | 3724353 A1 | 21-10-2020 |
| | | | WO | 2019117714 A1 | 20-06-2019 |
| WO 2015179493 | A1 | 26-11-2015 | US | 2017233727 A1 | 17-08-2017 |
| | | | WO | 2015179493 A1 | 26-11-2015 |
| WO 2015184016 | A2 | 03-12-2015 | EP | 3149168 A2 | 05-04-2017 |
| | | | US | 2017267997 A1 | 21-09-2017 |
| | | | WO | 2015184016 A2 | 03-12-2015 |
| WO 2020180659 | A1 | 10-09-2020 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9388456 B
- US 9695468 B
- US 20160350394 A1

**Non-patent literature cited in the description**

- **CHEN et al.** UMI-count modeling and differential expression analysis for single-cell RNA sequencing. *Genome Biology,* 2018
- **TAMBE et al.** *BMC Bioinformatics,* 2019
- **TAKENAKA et al.** Perfect Hamming code with a hash table for faster genome mapping. *BMC Bioinformatics,* 2011

```
                        SEQUENCE LISTING

   <110> Miltenyi Biotec B.V. & Co. KG

   <120> Algorithmic Method for Efficient Indexing of Genetic Sequences Using
   Associative Arrays

   <130> MBG_55

   <140> EP20207530.5
   <141> 2020-11-13

   <160> 2

   <170> BiSSAP 1.3.6

   <210> 1
   <211> 23
   <212> DNA
   <213> Artificial Sequence


   <220>
   <223> 5'end of padlock in Fig 3

   <400> 1
   ccagctcgta tgccgtcgtc tgc                                          23


   <210> 2
   <211> 23
   <212> DNA
   <213> Artificial Sequence


   <220>
   <223> 3' end of the padlock in Fig 3

   <400> 2
   gcctccacga cgctcctccg ctc                                          23
```